(19) Europäisches Patentamt
European Patent Office
Office européen des brevets

(11) **EP 2 448 527 B1**

(12) **EUROPÄISCHE PATENTSCHRIFT**

(45) Veröffentlichungstag und Bekanntmachung des Hinweises auf die Patenterteilung:
**24.06.2015 Patentblatt 2015/26**

(21) Anmeldenummer: **10730713.4**

(22) Anmeldetag: **28.06.2010**

(51) Int Cl.:
*A61F 2/66* (2006.01)          *A61F 2/68* (2006.01)
*A61F 2/50* (2006.01)          *A61F 2/70* (2006.01)
*A61F 2/76* (2006.01)

(86) Internationale Anmeldenummer:
**PCT/EP2010/003925**

(87) Internationale Veröffentlichungsnummer:
**WO 2011/000542 (06.01.2011 Gazette 2011/01)**

(54) **AKTIVE PROTHESENVORRICHTUNG MIT TERRAINERFASSUNG UND VERFAHREN ZUM STEUERN EINER AKTIVEN PROTHESENVORRICHTUNG**

ACTIVE PROSTHETIC DEVICE COMPRISING TERRAIN DETERMINATION MEANS AND METHOD FOR CONTROLLING SUCH A DEVICE

DISPOSITIF PROSTHÉTIQUE ACTIF DÉTECTANT LE TERRAIN ET PROCÉDÉ DE COMMANDE D UN DISPOSITIF PROSTHÉTIQUE ACTIF

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **30.06.2009 DE 102009030995**

(43) Veröffentlichungstag der Anmeldung:
**09.05.2012 Patentblatt 2012/19**

(73) Patentinhaber: **Fraunhofer-Gesellschaft zur Förderung der angewandten Forschung e.V.
80686 München (DE)**

(72) Erfinder:
• **SCHNEIDER, Urs
70563 Stuttgart (DE)**

• **KLEINER, Bernhard
70199 Stuttgart (DE)**
• **VON ROSENBERG, Harald
70569 Stuttgart (DE)**
• **BUDAKER, Bernhard
71336 Waiblingen (DE)**

(74) Vertreter: **Thum, Bernhard
Wuesthoff & Wuesthoff
Patentanwälte PartG mbB
Schweigerstraße 2
81541 München (DE)**

(56) Entgegenhaltungen:
**WO-A1-00/74610     US-A1- 2007 050 047**

**Beschreibung**

Die vorliegende Erfindung betrifft eine Prothesenvorrichtung, insbesondere Fußprothese oder Beinprothese, umfassend:

- einen Prothesenschaft, der mit einem zu versorgenden Bein eines Patienten koppelbar ist,
- wenigstens einen Auftrittkörper zum Kontaktieren eines Untergrunds, und
- wenigstens eine den Prothesenschaft mit dem Auftrittkörper gelenkig verbindende Gelenkeinrichtung,

wobei die Gelenkeinrichtung eine Aktorik aufweist, die mittels einer Steuereinrichtung derart aktiv ansteuerbar ist, dass die Lage des Auftrittkörpers relativ zum Prothesenschaft veränderbar ist.

Derartige Prothesenvorrichtungen sind aus dem Stand der Technik bekannt. So beschreibt beispielsweise das Dokument WO 2006/083913 A2 eine solche Prothesenvorrichtung, die als Fußprothese ausgebildet ist. Der Auftrittkörper, der der Gestalt eines menschlichen Fußes nachempfunden ist, weist eine Reihe von mechanischen Sensoren auf, die während dem Auftreten des Auftrittkörpers auf dem Untergrund je nach mechanischer Belastungssituation und Untergrundbeschaffenheit unterschiedlich stark mit Kräften und Momenten beaufschlagt werden. Die Sensoren sind derart angeordnet, dass sie sowohl die Intensität als auch Belastungsrichtungen erfassen können. Insbesondere sind die Sensoren als biegempfindliche Messstreifen, beispielsweise die Dehnmessstreifen oder dergleichen, ausgebildet. Die von den Sensoren erfassten Belastungsparameter werden in Echtzeit ausgewertet. Nach Maßgabe der aktuellen Auswertung wird die Aktorik der Gelenkeinrichtung angesteuert, um den Auftrittkörper der Prothesenvorrichtung situationsgerecht zu positionieren und zu fixieren.

Vergleichbare Lösungen, bei denen die aktuelle mechanische Belastungssituation des Auftrittkörpers infolge des Kontakts mit dem Untergrund durch Ermitteln von auftretenden Kräften und Momenten erfasst wird und anhand dieser Erfassung mehr oder weniger in Echtzeit die Aktorik der Gelenkeinrichtung angesteuert wird, sind darüber hinaus beispielsweise aus den Dokumenten US 2009/0069898 A1, US 6,755,870, EP 1 933 775 A2, EP 1 942 843 A2 oder EP 1 531 767 B1 bekannt.

Den in diesen Dokumenten offenbarten Gegenständen ist aber gemein, dass eine Erfassung hinsichtlich der Beschaffenheit des Untergrunds beziehungsweise Terrains, der beziehungsweise das gerade mit der Fußprothese begangen wird, erst dann erfolgt, wenn mechanischer Kontakt zwischen dem Auftrittkörper und dem Untergrund besteht. Ein Übergang von einer Untergrundart zu einer anderen Untergrundart wird daher verhältnismäßig spät erfasst. Anders als bei einer gesunden Person, die unterbewusst die Fußstellung auf die aktuell erkannte Untergrundbeschaffenheit einstellt, bevor der Fuß aufgesetzt wird, benötigen die Fußprothesen gemäß dem Stand der Technik also unmittelbaren Kontakt mit dem Untergrund. Mit anderen Worten werden die Fußprothesen gemäß diesem Stand der Technik auf einen solchen Übergang in der Beschaffenheit des Untergrunds erst relativ spät eingestellt, was bei dem Patienten zu einem unsicheren Gefühl führen oder schlimmstenfalls sogar der Sturzrisiko erheblich vergrößern kann. Insbesondere beim Begehen von Hindernissen, wie Türschwellen, Treppen oder Schrägen können mit herkömmlichen Prothesenvorrichtungen erhebliche Probleme auftreten.

Das Dokument EP 2 036 518 A1 beschreibt eine Anordnung mit einer verhältnismäßig aufwändigen Mechanik, die darauf abzielt, die Trägheitsmomente zu reduzieren und damit die Gesamtbelastung auf das Bein des Benutzers zu reduzieren.

Es ist daher Aufgabe der vorliegenden Erfindung, eine Prothesenvorrichtung der eingangs bezeichneten Art sowie ein Verfahren zum Steuern einer solchen Prothesenvorrichtung bereitzustellen, mit der bereits frühzeitig eine Erfassung des begangenen Terrains oder Untergrunds unter Vermeidung vorstehend beschriebener Probleme möglich ist.

Diese Aufgabe wird mit einer Prothesenvorrichtung der eingangs bezeichneten Art gelöst, bei der vorgesehen ist, dass die Prothesenvorrichtung wenigstens ein Sensorelement aufweist, das zur berührungsfreien Erfassung der Beschaffenheit des Untergrunds ausgebildet ist, wobei die Aktorik der Gelenkeinrichtung nach Maßgabe der von dem Sensorelement hinsichtlich der Beschaffenheit des Untergrunds berührungslos erfassten Information ansteuerbar ist.

Mit anderen Worten ist bei der erfindungsgemäßen Prothesenvorrichtung eine Erfassung des begangenen Terrains oder Untergrunds bereits zu einem Zeitpunkt möglich, zudem noch gar kein Kontakt des Auftrittkörpers mit dem Untergrund besteht. Die Auftrittkörper befindet sich sozusagen noch in einer erhabenen Position entfernt von und über dem Untergrund und kann in dieser Position berührungsfreien die Beschaffenheit des Untergrunds, insbesondere dessen dreidimensionales Profil mit etwaigen Unebenheiten, Schrägen, Löchern etc., erfassen und in der Folge die Aktorik der Prothesenvorrichtung gezielt auf dieses Profil einstellen. Ebenso wie beim Stand der Technik erfolgt auch bei der Erfindung eine Echtzeitauswertung der erfassten Information, so dass das Verhalten der erfindungsgemäßen Prothesenvorrichtung auf-grund der Einstellung der Aktorik in einem frühen Stadium vor dem Kontaktieren des Untergrunds mit dem Auftrittkörper dem unterbewussten Verhalten einer gesunden Person nahezu ideal angenähert wird.

Unter dem Begriff "Prothesenvorrichtung" im Zusammenhang mit der Erfindung sind reine Fußprothesen, aber auch Beinprothesen zu verstehen, wobei letztere neben der Gelenkeinrichtung am Auftrittkörper auch mit zusätzlichem Gelenk, beispielsweise Kniegelenk, ausgeführt sein können. Der Begriff "Prothesenschaft" umfasst die gesamte Verbindungsanordnung zum Patienten, gegebenenfalls auch mehrteilig mit passivem oder aktivem Kniegelenk ausgeführt.

Bei einer bevorzugten Ausführungsvariante der Erfindung kann vorgesehen sein, dass das Sensorelement eine Kamera oder einen Distanzmesssensor aufweist. Derartige Sensoren können akustische Sensoren, insbesondere Ultraschallsensoren, optische Sensoren, scannende Sensoren oder dergleichen sein. Als Kameras kommen einfache Kamerasensoren, aber auch 3D-Kamerasensoren, beispielsweise PMDs, infrage.

In diesem Zusammenhang ist es erfindungsgemäß möglich, dass an dem Auftrittkörper eine Mehrzahl von Sensorelementen angeordnet ist. Durch die Anbringung einer Mehrzahl von Sensorelementen lässt sich ein größerer Bereich des Untergrunds beziehungsweise begangenen Terrains erfassen und auswerten. So ist es dadurch auch möglich, bestimmte Felder des Untergrunds mit mehreren, gegebenenfalls auch mit mehreren verschiedenartigen, Sensorelementen zu erfassen, um so verlässlichere Information über die Beschaffenheit des Untergrunds zu erhalten und mögliche Fehlerfassungen ausschließen zu können.

Bei einer bevorzugten Variante der Erfindung ist vorgesehen, dass jeweils zwei Sensorelemente, insbesondere jeweils zwei Kameras, zur Erfassung eines Stereobildes des Untergrunds kombinierbar sind. Anhand des Stereobildes lässt sich einfach mit herkömmlichen Bildverarbeitungstechniken ein Tiefenbild des Untergrunds beziehungsweise begangenen Terrains ermitteln, auf dessen Profil abgestimmt sich dann die Aktorik der Gelenkeinrichtung ansteuern und der Auftrittkörper der Prothesenvorrichtung positionieren lässt.

Üblicherweise wird der Auftrittkörper von solchen Prothesenvorrichtungen der Form eines menschlichen Fußes nachempfunden. Bei einer derartigen Gestalt aber auch bei hiervon abweichend ausgebildeten Auftrittkörpern kann erfindungsgemäß vorgesehen sein, dass an dem Auftrittkörper an dessen dem Untergrund zugewandter Sohle eine Mehrzahl von Sensorelementen angeordnet ist. Gerade die dem Untergrund zugewandte Sohle bietet eine verhältnismäßig große Fläche, an der an verschiedenen Stellen entsprechende Sensorelemente angebracht werden können, um so ein möglichst großes Feld des begangenen Untergrunds erfassen zu können. Dabei können die Sensorelemente in schützenden Ausnehmungen in der Sohle angeordnet sein. Sie können durch entsprechende optisch und akustisch transparente Abdeckungen vor äußeren Einflüssen geschützt werden, wie beispielsweise vor Feuchtigkeit, Schmutz oder mechanischer Beschädigung.

Zusätzlich oder alternativ zu einer Anbringung der Sensorelemente an der Sohle kann erfindungsgemäß vorgesehen sein, dass an dem Auftrittkörper stirnseitig oberhalb der dem Untergrund zugewandten Sohle eine Mehrzahl von Sensorelementen angeordnet ist. So ist es möglich, Sensorelemente an einem dem menschlichen Fuß nachempfunden Auftrittkörper im Bereich der Zehen oder/und im Bereich der Ferse anzuordnen. Durch diese Maßnahme lassen sich die Sensorelemente einerseits geschützt gegen im Auftrittbereich drohende mechanische Einwirkungen und andererseits in einer Lage anbringen, die eine vorausschauende Untergrunderfassung ermöglicht.

Anders als bei der eingangs beschriebenen mechanischen Erfassung der Untergrundbeschaffenheit, die anhand der aktuell auf den Auftrittkörper wirkenden Kräfte im Falle eines Kontakts mit dem Untergrund erfolgt, sieht die Erfindung eine berührungsfreie Untergrunderfassung vor. Um die Qualität der Erfassung des dreidimensionalen Untergrundprofils zu verbessern, sieht eine Ausführungsform der Erfindung vor, dass eine Projektionseinrichtung an dem Auftrittkörper angeordnet ist, mittels der ein optisch erfassbares Muster, insbesondere ein Gitter, auf den Untergrund projizierbar ist, wobei anhand der Form, d.h. dem Bild, des auf den Untergrund projizierten optisch erfassbaren Musters die Beschaffenheit des Untergrunds ermittelbar ist. Im Falle von Unebenheiten, Neigungen oder Steigungen sowie Gefällen des begangenen Untergrunds wird gemäß dieser Ausführungsvariante der Erfindung beispielsweise ein engmaschiges Gitter mit aufeinander senkrecht stehenden äquidistanten Gitterlinien durch die Unebenheiten etc. bei der Projektion auf den Untergrund verworfen, so dass sich auf dem Untergrund ein unregelmäßiges Bild des Gitters ergibt. Wird nun dieses Bild des Gitters auf dem Untergrund erfasst, beispielsweise mittels vorstehend genannter Kameras, so kann mit herkömmlichen Bildverarbeitungstechniken in Echtzeit verhältnismäßig exakt das tatsächliche Profil des Untergrunds berechnet und darauf basierend die Aktorik der Gelenkeinrichtung angesteuert werden.

Erfindungsgemäß kann die Prothesenvorrichtung ferner mit wenigstens einer Bewegungserfassungseinrichtung ausgebildet sein, die zur Erfassung von Bewegungsparametern bezüglich der aktuellen Bewegung der Prothesenvorrichtung, insbesondere-bezüglich einer Rotation und Translation der Prothesenvorrichtung, ausgebildet ist. In diesem Zusammenhang kann vorgesehen sein, dass die Bewegungserfassungseinrichtung wenigstens einen Rotationssensor oder/und wenigstens einen Trägheitssensor aufweist. Darüber hinaus können auch Magnetfeldsensoren, Beschleunigungssensoren oder Drehratensensoren eingesetzt werden. Mit der Bewegungserfassungseinrichtung lässt sich die aktuelle translatorische sowie rotatorische Bewegung der Fußprothese im Raum anhand von sechs Freiheitsgraden (drei translatorische und drei rotatorische bezüglich der Raumachsen) ermitteln.

Zur kompakten Ausgestaltung der erfindungsgemäßen Prothesenvorrichtung kann gemäß einer weiteren Ausführungsvariante der Erfindung vorgesehen sein, dass die Bewegungserfassungseinrichtung an dem Schaft der Prothesenvorrichtung angeordnet oder in diesem integriert ist. Sie kann beispielsweise in einem Gehäuse angeordnet sein, die am oder im Schaft ausgebildet ist.

Eine Weiterbildung der Erfindung sieht vor, dass die Steuereinrichtung dazu ausgebildet ist, die von der Bewegungserfassungseinrichtung erfassten Bewegungsparameter in Kombination mit der von dem wenigstens einen Sensorelement erfassten Information bezüglich der Beschaffenheit des Untergrunds auszuwerten, um ein Modell des Untergrunds zu ermitteln, nach Maßgabe dessen die Aktorik der Prothesenvorrichtung einstellbar ist. Mit anderen Worten wird anhand der Bewegungsparameter die aktuelle Position der Fußprothese im Raum bezüglich eines globalen Koordinatensystems ermittelt und diesem globalen Koordinatensystem dann die erfassten Information über den Untergrund und dessen Beschaffenheit zugeordnet. Dadurch lässt sich gewährleisten, dass die Prothesenvorrichtung in Echtzeit auf das Profil des Untergrunds und dessen Beschaffenheit in genau dem Bereich abgestimmt wird, in dem der Auftrittkörper als nächstes im Zuge des gerade ausgeführten Schrittes auf den Untergrund auftrifft und den Patienten stützt.

Vorstehend wurde bereits angedeutet, dass erfindungsgemäß vorgesehen sein kann, bestimmte Untergrundbereiche sozusagen mehrfach durch verschiedene Sensorelemente zu erfassen. Die über die Beschaffenheit des jeweiligen Untergrundbereichs von den verschiedenen Sensorelementen gesammelte Information lässt sich dann auswerten und miteinander vergleichen. Im Falle einer wesentlichen Übereinstimmung kann darauf geschlossen werden, dass die von den Sensorelementen gelieferten Informationen zutreffend sind und die Beschaffenheit des Untergrunds korrekt ermittelt wurde. Im Falle starker Abweichungen hinsichtlich der über den untersuchten Untergrundbereich von den verschiedenen Sensorelementen gesammelten Informationen kann auf eine Fehlerfassung geschlossen werden. In diesem Zusammenhang kann erfindungsgemäß vorgesehen sein, dass die erfindungsgemäße Prothesenvorrichtung mit wenigstens einem Fusionsfilter ausgebildet ist, dem zur Plausibilitätskontrolle Signale zuführbar sind, die die bezüglich des Untergrunds durch das wenigstens eine Sensorelement ermittelte Information sowie die Bewegungsparameter der Bewegungserfassungseinrichtung repräsentieren.

Die Erfindung betrifft ferner ein Verfahren zum Steuern bzw. Regeln einer Prothesenvorrichtung, insbesondere nach einem der vorangehenden Ansprüche, wobei die Prothesenvorrichtung umfasst:

- einen Prothesenschaft, der mit einem zu versorgenden Bein eines Patienten koppelbar ist,
- wenigstens einen Auftrittkörper zum Kontaktieren eines Untergrunds, und
- wenigstens eine den Prothesenschaft mit dem Auftrittkörper gelenkig verbindende Gelenkeinrichtung,

wobei die Gelenkeinrichtung eine Aktorik aufweist, die mittels einer Steuereinrichtung derart aktiv ansteuerbar ist, dass die Lage des Auftrittkörpers relativ zum Prothesenschaft veränderbar ist, wobei die Prothesenvorrichtung wenigstens ein Sensorelement aufweist, das zur berührungsfreien Erfassung der Beschaffenheit des Untergrunds ausgebildet ist, umfassend die Schritte:

- berührungsfreies Erfassen der Beschaffenheit des Untergrunds mittels des wenigstens einen Sensorelements,
- Auswerten der von dem wenigstens einen Sensorelement bereitgestellten Information hinsichtlich der Beschaffenheit des Untergrunds und
- Ansteuern der Aktorik der Gelenkeinrichtung nach Maßgabe der von dem Sensorelement hinsichtlich der Beschaffenheit des Untergrunds ausgegebenen Information.

Wie vorstehend bereits ausgeführt, kann auch bei dem erfindungsgemäßen Verfahren vorgesehen sein, dass die Prothesenvorrichtung eine Projektionseinrichtung aufweist, wobei mit der Projektionseinrichtung ein optisch erfassbares Muster, insbesondere ein Gitter, auf den Untergrund projiziert wird, wobei die Istform, das heißt das Bild, des auf den Untergrund projizierten optischen Musters erfasst wird und mit einer Sollform verglichen wird und anhand der Abweichung zwischen der Istform und der Sollform die Beschaffenheit des Untergrunds, insbesondere dessen Geometrie, ermittelt wird.

Ferner kann bei dem erfindungsgemäße Verfahren vorgesehen sein, dass die Prothesenvorrichtung eine Bewegungserfassungseinrichtung aufweist, die Bewegungsparameter bezüglich der aktuellen Bewegung der Prothesenvorrichtung, insbesondere bezüglich einer Rotation und Translation der Prothesenvorrichtung, erfasst.

Eine Weiterbildung des erfindungsgemäßen Verfahrens sieht vor, dass die von der Bewegungserfassungseinrichtung erfassten Bewegungsparameter in Kombination mit der von dem wenigstens einen Sensorelement erfassten Information bezüglich der Beschaffenheit des Untergrunds ausgewertet werden, dass anhand des Auswertungsergebnisses ein Modell, insbesondere ein dreidimensionales Modell, des Untergrunds ermittelt wird und dass nach Maßgabe des ermittelten Modells des Untergrunds die Aktorik der Prothesenvorrichtung angesteuert wird.

Dabei kann bei einer Weiterbildung der Erfindung vorgesehen sein, dass die von dem wenigstens einen Sensorelement erfasste Information in Relation mit den von der Bewegungserfassungseinrichtung erfassten Bewegungsparametern gebracht wird, derart, dass sie in ein globales Bezugssystem transformiert wird. Insbesondere kann hierbei vorgesehen sein, dass die Transformation in das globale Bezugssystem mittels Quaternionenrotation erfolgt.

Die Transformation erfolgt anhand der folgenden Beziehung:

$$X = X_{pos} + D \cdot \begin{bmatrix} (q_0^2 + q_1^2 - q_2^2 - q_3^2) + 2(q_1 q_2 - q_0 q_3) + 2(q_1 q_3 + q_0 q_2) \\ 2(q_1 q_2 + q_0 q_3) + (q_0^2 - q_1^2 + q_2^2 - q_3^2) + 2(q_2 q_3 + q_0 q_1) \\ 2(q_1 q_3 - q_0 q_2) + 2(q_2 q_3 + q_0 q_1) + (q_0^2 + q_1^2 - q_2^2 + q_3^2) \end{bmatrix}$$

wobei

$X$       dreidimensionale Lageinformation eines Strukturpunktes im globalen Koordinatensystem darstellt,

$X_{pos}$     dreidimensionale Positionsinformation des Messsystems im globalen Koordinatensystem darstellt,

$D$       die Distanz zwischen Messsystem und gemessenem Strukturpunkt ist, und

$q_1...q_n$    Quaternionen sind, welche die dreidimensionale Orientierung des Messsystems im Raum repräsentieren.

Eine Weiterbildung des erfindungsgemäßen Verfahrens sieht vor, dass eine Plausibilitätskontrolle hinsichtlich der über eine Mehrzahl von Sensorelementen ermittelten Informationen über die Beschaffenheit des Untergrunds durchgeführt wird, wobei nicht-plausible Informationen verworfen werden. Dieses Verfahrensmerkmal wurde vorstehend bereits hinsichtlich der erfindungsgemäßen Prothesenvorrichtung erörtert.

Bei dem erfindungsgemäßen Verfahren kann ferner vorgesehen sein, dass bei der Ermittlung der Beschaffenheit des Untergrunds Navigationsdaten eines Navigationssystems berücksichtigt werden. So kann auch anhand der Navigationsdaten auf die Beschaffenheit des Untergrunds, insbesondere des Untergrundprofils geschlossen werden, weil sich aus den Navigationsdaten üblicherweise auch Hinweise über Gefälle, Bodenbeschaffenheit etc. ergeben können.

Die Erfindung wird im Folgenden beispielhaft anhand der beiliegenden Figuren erläutert. Es stellen dar:

Fig. 1     eine schematische Ansicht einer erfindungsgemäßen Fußprothese;

Fig. 2     eine Ansicht auf die Sohle eines Auftrittkörpers, der dem menschlichen Fuß nachempfunden ist; und

Fig. 3     ein Blockschaltbild zur Erläuterung der Komponenten und Funktionsweise der erfindungsgemäßen Fußprothese.

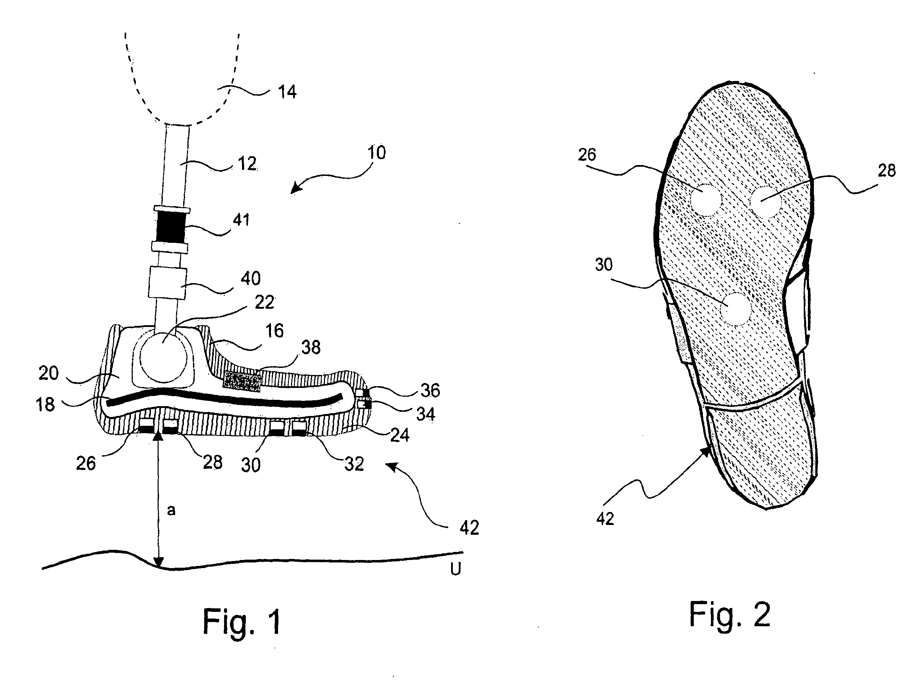

In Figur 1 ist eine erfindungsgemäße Prothesenvorrichtung, in diesem speziellen Ausführungsbeispiel ausgeführt als Fuß- bzw. Beinprothese, in schematischer Darstellung gezeigt und allgemein mit 10 bezeichnet. Diese umfasst einen Prothesenschaft 12, der in nicht näher gezeigter Weise an einem Beinabschnitt 14 eines mit der Prothesenvorrichtung 10 zu versorgenden Patienten koppelbar ist. Am patientenfernen Ende der Fußprothese 10 ist ein Auftrittkörper 16 angebracht, der in seiner Form der Gestalt eines menschlichen Fußes nachempfunden ist.

Der Auftrittkörper umfasst eine flexible aber dennoch stabile Auftrittplatte 18, wie allgemein bekannt, die mit einem Kunststoffmaterial 20 umhüllt ist. Der Auftrittkörper 16, insbesondere die Auftrittplatte 18, ist über eine aktive Gelenkeinrichtung 22 mit dem Schaft 12 verbunden. Die Gelenkeinrichtung 22 umfasst eine in Fig. 1 nicht näher gezeigte Aktorik, mit der sich nach Maßgabe von Steuersignalen der Auftrittkörper 16 relativ zum Prothesenröhr 12 verlagern lässt. Man erkennt, dass der Auftrittkörper 16 mit einem Schuh ausgebildet ist. Der Schuh umfasst in herkömmlicher Weise eine Sohle 24, beispielsweise aus Gummi, die sich über den Frontbereich und den Fersenbereich erstreckt. Die Sohle 24 ist verhältnismäßig dick ausgebildet. In der Sohle 24 sind einzelne Sensorelemente 26, 28, 30, 32, 34 so angeordnet, dass sie vor äußeren Einflüssen, insbesondere vor Nässe, mechanischen Einwirkungen sowie Verschmutzung geschützt sind. Das Sensorelement 34 ist als Kamera ausgebildet, wohingegen die Sensorelemente 26, 38, 30 und 32 als Distanzmesssensoren ausgebildet sind. Die Komponente 36 ist als Projektor ausgeführt, mit dem sich ein Lichtgitter auf den Untergrund projizieren lässt, dessen Bild dann von der Kamera 34 erfasst wird. Am Prothesenschaft beziehungsweise Prothesenrohr 12 ist ferner ein Laserscanner 41 vorgesehen, der zweidimensionale Distanzinformationen durch Abscannen der Umgebung erfasst und der Steuereinrichtung 38 bereitstellt.

Die Sensorelemente 26 bis 36 sind mit einer Steuereinrichtung 38 gekoppelt, die zur Ansteuerung der Aktorik in der Gelenkeinrichtung 22 dient. Die Steuereinrichtung 38 ist in den Auftrittskörper 16 integriert. Ferner ist in das Prothesenrohr 12 eine Bewegungserfassungseinrichtung 40 integriert, die mit einer Sensorik zur Erfassung der aktuellen Bewegung der Prothesenvorrichtung 12 im Raum ausgebildet ist. Hierzu sind Sensoren, insbesondere Magnetfeldsensoren, Drehratensensoren sowie Beschleunigungssensoren geeignet. Mit der Bewegungserfassungseinrichtung 40 lassen sich translatorische und rotatorische Bewegungen der Fußprothese erfassen und an die Steuereinrichtung 38 ausgeben. Insbesondere ist eine Bewegungserfassung hinsichtlich aller sechs Freiheitsgrade möglich, das heißt translatorische Bewegungen entlang den Raumachsen sowie Drehbewegungen um die Raumachsen.

Fig. 2 zeigt eine zu der Gestaltung des Auftrittkörpers alternative Gestaltung, bei der lediglich drei Sensorelemente 26, 28 und 30 an der Sohle eines den Auftrittkörper umgebenden Schuhs 42 angeordnet sind.

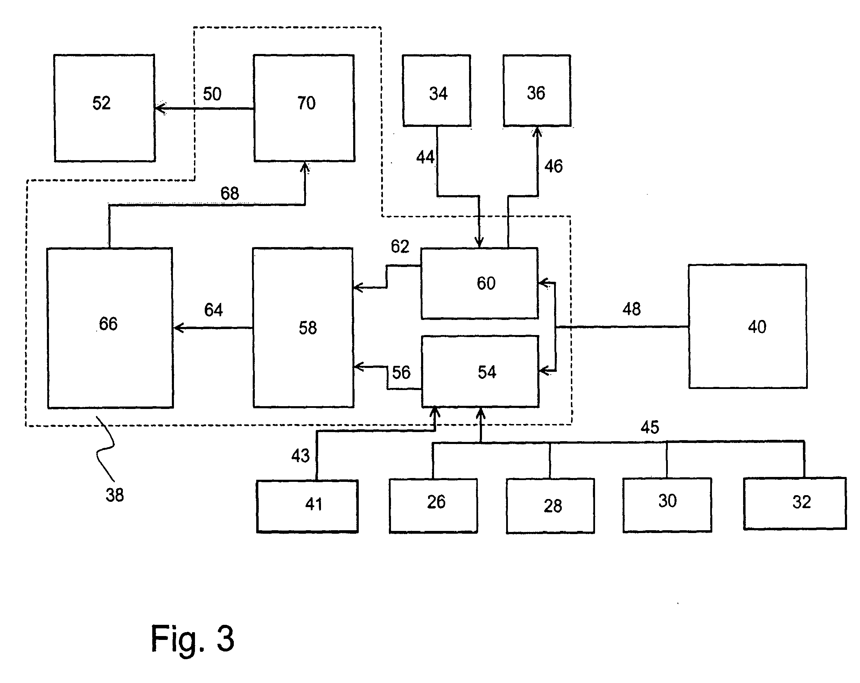

Mit Bezug auf Fig. 3 sollen der Aufbau und die Funktionsweise der erfindungsgemäßen Prothesenvorrichtung 10 im Detail erläutert werden. Man erkennt die Steuereinrichtung 38 schematisch dargestellt durch eine Reihe von Funktionsblöcken. Mit der Steuereinrichtung 38 sind die Kamera 34, die Distanzsensoren 26, 28, 30 und 32 sowie der Projektor 36 verbunden. Die Kamera 34 liefert in Echtzeit einen permanenten Strom an Videodaten über die Signalleitung 44, die von ihr von dem Untergrund aufgenommen wurden. Der Projektor 36 wird über eine Signalleitung 46 mit einem Steuersignal versorgt. Die Distanzsensoren 26, 28, 30, 32 liefern über die Signalleitung 45 regelmäßig Informationen über den Abstand a zwischen der Sohle des Auftrittkörpers 16 und dem Untergrund U (siehe Fig. 2). Der Laserscanner 41 liefert über die Signalleitung 43 permanent zweidimensionale Distanzinformationen bezüglich einzelner Objekte der Umgebung.

Ferner ist an die Steuereinrichtung die Bewegungserfassungseinrichtung 40 angeschlossen. Diese liefert permanent Daten über die aktuelle Bewegung der erfindungsgemäßen Fußprothese 10 im Raum über die Signalleitung 48.

Die Steuereinrichtung 38 steuert mit entsprechenden Steuersignalen über den Signalpfad 50 die Aktorik 52 der Prothesenvorrichtung 10 an. Nach Maßgabe dieser Steuersignale werden verschiedene Stellglieder der Aktorik 52 betätigt, um so den Auftrittkörper 16 der Prothesenvorrichtung 10 relativ zum Prothesenschaft 12 einzustellen.

Die Steuereinrichtung 38 weist die folgenden Komponenten auf und funktioniert wie folgt:

Die von den Distanzsensoren 26, 28, 30, 32 bereitgestellten Informationen über den Abstand a zwischen dem Auftrittkörper 16 und dem Untergrund U sowie die vom Laserscanner 41 bereitgestellten zweidimensionalen Distanzinformationen zum Untergrund U werden zusammen mit den von der Bewegungserfassungseinrichtung 40 bereitgestellten Daten einem Strukturextraktionsblock 54 zugeführt. Dieser ermittelt anhand der erhaltenen Daten von den Distanzsensoren 26, 28, 30, 32, dem Laserscanner 41 und von der Bewegungserfassungseinrichtung 40 Daten zu einem dreidimensionalen Modell des Untergrunds beziehungsweise begangenen Terrains und gibt diese dreidimensionalen Umgebungsdaten über die Signalleitung 56 an einen Umgebungsextraktionsblock 58 aus, der diese Daten in nachfolgend näher beschriebener Weise auswertet.

Die von der Kamera 34 bereitgestellten Videodaten werden über die Signalleitung 44 zusammen mit den von der Bewegungserfassungseinrichtung 40 bereitgestellten Daten einem Funktionsblock 60 zugeführt, der eine bildbasierte Strukturextraktion durchführt. Dieser Funktionsblock 60 gibt ferner das Steuersignal an den Projektor 36 über die Signalleitung 46 aus, damit der Projektor 36 ein Gitter auf den Untergrund projiziert, dessen Bild wiederum von der Kamera 34 erfasst werden kann. Der Funktionsblock 60 zur bildbasierten Strukturextraktion gibt über die Signalleitung 62 dreidimensionale Umgebungsdaten an den Umgebungsextraktionsblock 58 aus.

In dem Umgebungsextraktionsblock 58 werden die dreidimensionalen Umgebungsdaten vom Funktionsblock 54, das heißt diejenigen Umgebungsdaten, die anhand der Signale von dem Distanzsensoren 26, 28, 30, 32 sowie Laserscanner 41 ermittelt wurden, mit denjenigen dreidimensionalen Umgebungsdaten zusammengeführt, die aus der bildbasierten Strukturextraktion des Funktionsblocks 60 ermittelt wurden. Bei dieser Zusammenführung beziehungsweise Sensordatenfusion wird eine Plausibilitätskontrolle durchgeführt, das heißt es wird erfasst, ob einzelne Umgebungsdaten möglicherweise von der Kamera 34 oder einem der Distanzsensoren 26, 28, 30, 32 sowie des Laserscanners 41 oder im Rahmen der jeweiligen Strukturextraktion fehlerhaft ermittelt wurden. Fehlerhaft ermittelte Umgebungsdaten werden verworfen. Nach der Fusion der ermittelten dreidimensionalen Umgebungsdaten im Funktionsblock 58 gibt dieser über die Signalleitung 64 fusionierte Umgebungsdaten aus, das heißt auf Plausibilität überprüfte Umgebungsdaten, die sowohl die Untergrunderfassung über die Distanzsensoren 26, 28, 30, 32 sowie über den Laserscanner 41 als auch die Untergrunderfassung über die Kamera 34 berücksichtigen.

Die über die Signalleitung 64 ausgegebenen fusionierten Umgebungsdaten werden dem Funktionsblock 66 zugeführt, der der Objekterkennung beziehungsweise Hinderniserkennung dient. Dieser Funktionsblock 66 ist dazu ausgebildet, aus den fusionierten Umgebungsdaten Hindernisse beziehungsweise Objekte zu erkennen, die auf Unregelmäßigkeiten im Untergrund U schließen lassen, wie beispielsweise Neigungen, Schrägen, Kanten, Stufen, Barrieren, Unebenheiten etc., und die eine besondere Einstellung der Fußprothese 10, insbesondere des Auftrittkörpers 16 oder eines Kniegelenks (andere Ausführung) relativ zum Prothesenrohr 12 erfordern. Der Funktionsblock 66 dient demnach dazu, hinsichtlich dem Verhalten der Fußprothese das menschliche Unterbewusstsein zu imitieren, das bei einer gesunden Person regelmäßig den begangenen Untergrund erfasst und mögliche Objekte erkennt, und in der Folge das Fußgelenk entsprechend einstellt, um den Untergrund mit diesen Objekten sicher und sturzfrei zu begehen.

Von dem Funktionsblock 66 zur Erkennung von Objekten beziehungsweise Hindernissen werden über die Signalleitung 68 Objektinformationen bereitgestellt. Diese Objektinformationen werden in dem Funktionsblock 70 verarbeitet, der der Prothesensteuerung dient. Nach Maßgabe der Objektinformationen generiert der Funktionsblock 70 zur Prothesensteuerung Steuersignale, die über die Signalleitung 50 an die Aktorik 52 der Prothese ausgegeben werden. Wie bereits ausgeführt, wird die Aktorik 52 über diese Steuersignale derart angesteuert, dass der Auftrittkörper 16 relativ zum Schaft 12 in einer Weise positioniert wird, die das unterbewusste Verhalten eines gesunden Menschen bezüglich der Positionierung des Fußgelenks nachempfindet.

Mit der erfindungsgemäßen Fußprothese 10 der vorstehend beschriebenen Art ist es möglich, frühzeitig, das heißt lange vor einem unmittelbaren mechanischen Kontakt mit dem begangenen Untergrund, etwaige Hindernisse oder auf dem Untergrund befindliche Objekte, Unregelmäßigkeiten etc. in Echtzeit zu erkennen und die Fußprothese auf diese besonderen Untergrundbeschaffenheiten einzustellen, bevor tatsächlich ein Kontakt der Fußprothese mit dem Untergrund erfolgt. Anders als beim Stand der Technik ist die aktiv ansteuerbare erfindungsgemäße Fußprothese daher bereits vor und bei dem Aufsetzen auf den Untergrund auf die im Auftrittbereich vorhandene Untergrundbeschaffenheit, insbesondere dessen Profil, vorbereitet und eingestellt, was es einer mit einer solchen Fußprothese versorgten Person ermöglicht, auch unregelmäßig profiliertes Terrain erheblich sicherer und mit deutlich reduziertem Sturzrisiko zu begehen, als dies bei herkömmlichen erst im Kontaktfall aktiv eingreifenden Fußprothesen möglich ist.

Vorstehend wurde die Erfindung am Beispiel einer Fußprothese erläutert.

Es ist für den Fachmann auch selbstverständlich, dass sich vergleichbare Lösungen auch für andersartige Prothesen einsetzen lassen, beispielsweise für Beinprothesen, aber auch für Hand- oder Armprothesen. Wie bereits ausgeführt, kann bei Ausführung der Prothese als Beinprothese neben der Gelenkeinrichtung 22 auch eine weitere aktive Gelenkeinrichtung vorgesehen sein, beispielsweise ein aktiv ansteuerbares Kniegelenk (nicht gezeigt), die nach Maßgabe der berührungslos erfassten Informationen im Zusammenspiel mit der Ansteuerung der auftrittkörperseitigen Gelenkeinrichtung 22 ansteuerbar ist. Auch im Fall von Hand- oder Armprothesen ist eine frühzeitige Objekterkennung und Adaption der Prothese auf die jeweilige Bewegungs- oder Griffsituation äußerst vorteilhaft.

## Patentansprüche

1.  Prothesenvorrichtung (10), insbesondere Fußprothese oder Beinprothese, umfassend:

    - einen Prothesenschaft (12), der mit einem zu versorgenden Bein eines Patienten koppelbar ist,
    - wenigstens einen Auftrittkörper (16) zum Kontaktieren eines Untergrunds (U), und
    - wenigstens eine den Prothesenschaft (12) mit dem Auftrittkörper (16) gelenkig verbindende Gelenkeinrichtung (22),

    wobei die Gelenkeinrichtung (22) eine Aktorik (52) aufweist, die mittels einer Steuereinrichtung (38) derart aktiv ansteuerbar ist, dass die Lage des Auftrittkörpers (16) relativ zum Prothesenschaft (12) veränderbar ist, und die Prothesenvorrichtung (10) ferner wenigstens ein Sensorelement (26, 28, 30, 32, 34, 36, 41) aufweist, das zur berührungsfreien Erfassung der Beschaffenheit des Untergrunds ausgebildet ist, wobei die Aktorik (52) der Gelenkeinrichtung (22) nach Maßgabe der von dem Sensorelement (26, 28, 30, 32, 34, 36, 41) hinsichtlich der Beschaffenheit des Untergrunds (U) berührungslos erfassten Information ansteuerbar ist, sowie wenigstens eine Bewegungserfassungseinrichtung (40), die zur Erfassung von Bewegungsparametern bezüglich der aktuellen Bewegung der Prothesenvorrichtung (10), insbesondere bezüglich einer Rotation und Translation der Prothesenvorrichtung (10), ausgebildet ist, dadurch gekennzeichnet, dass die Steuereinrichtung (38) dazu ausgebildet ist, die von der Bewegungserfassungseinrichtung (40) erfassten Bewegungsparameter in Kombination mit der von dem wenigstens einen Sensorelement (26, 28, 30, 32, 34, 36, 41) erfassten Information bezüglich der Beschaffenheit des Untergrunds auszuwerten, um ein Modell des Untergrunds zu ermitteln, nach Maßgabe dessen die Aktorik (52) der Prothesenvorrichtung (10) einstellbar ist, wobei anhand der Bewegungsparameter die aktuelle Position der Prothesenvorrichtung (10) im Raum bezüglich eines globalen Koordinatensystems ermittelt und diesem globalen Koordinatensystem dann die erfasste Information über den Untergrund (11) und dessen Beschaffenheit zugeordnet wird.

2.  Prothesenvorrichtung (10) nach Anspruch 1, dadurch gekennzeichnet, dass das Sensorelement (26, 28, 30, 32, 34, 36, 41) eine Kamera oder einen Distanzmesssensor oder einen Laserscanner aufweist.

3.  Prothesenvorrichtung (10) nach Anspruch 1 oder 2, dadurch gekennzeichnet, dass an dem Auftrittkörper (16) eine Mehrzahl von Sensor-elementen angeordnet ist.

4.  Prothesenvorrichtung (10) nach einem der vorangehenden Ansprüche, dadurch gekennzeichnet, dass jeweils zwei Sensorelemente, insbesondere jeweils zwei Kameras, zur Erfassung eines Tiefenbildes des Untergrunds kombinierbar sind.

**5.** Prothesenvorrichtung (10) nach einem der vorangehenden Ansprüche,
**dadurch gekennzeichnet, dass** an dem Auftrittkörper (16) an dessen dem Untergrund zugewandten Sohle eine Mehrzahl von Sensorelementen (26, 28, 30, 32, 34, 36, 41) angeordnet ist.

**6.** Prothesenvorrichtung (10) nach einem der vorangehenden Ansprüche,
**dadurch gekennzeichnet, dass** an dem Auftrittkörper (16) stirnseitig oberhalb der dem Untergrund zugewandten Sohle eine Mehrzahl von Sensorelementen (26, 28, 30, 32, 34, 36, 41) angeordnet ist.

**7.** Prothesenvorrichtung (10) nach einem der vorangehenden Ansprüche, **gekennzeichnet durch** eine an dem Auftrittkörper (16) angeordnete Projektionseinrichtung (36), mittels der ein optisch erfassbares Muster, insbesondere ein Gitter, auf den Untergrund projizierbar ist, wobei anhand der Form des auf den Untergrund projizierten optisch erfassbaren Musters die Beschaffenheit des Untergrunds ermittelbar ist.

**8.** Prothesenvorrichtung (10) nach einem der vorangegangenen Ansprüche, **dadurch gekennzeichnet, dass** die Bewegungserfassungseinrichtung (40) wenigstens einen Rotationssensor oder/und wenigstens einen Trägheitssensor aufweist.

**9.** Prothesenvorrichtung (10) nach einem der vorangegangenen Ansprüche, **dadurch gekennzeichnet, dass** die Bewegungserfassungseinrichtung (40) an dem Prothesenschaft (12) oder dem Auftrittkörper (16) der Prothesenvorrichtung (10) angeordnet oder in diesem integriert ist.

**10.** Prothesenvorrichtung (10) nach einem der vorangegangenen Ansprüche, **gekennzeichnet durch** wenigstens ein Fusionsfilter, dem zur Plausibilitätskontrolle Signale zuführbar sind, die die bezüglich des Untergrunds **durch** das wenigstens eine Sensorelement (26, 28, 30, 32, 34, 36, 41) ermittelte Information sowie die Bewegungsparameter der Bewegungserfassungseinrichtung (40) repräsentieren.

**11.** Verfahren zum Steuern bzw. Regeln einer Prothesenvorrichtung (10), insbesondere nach einem der vorangehenden Ansprüche, wobei die Prothesenvorrichtung (10) umfasst:

- einen Prothesenschaft (12), der mit einem zu versorgenden Bein eines Patienten koppelbar ist,
- wenigstens einen Auftrittkörper (16) zum Kontaktieren eines Untergrunds, und
- wenigstens eine den Prothesenschaft (12) mit dem Auftrittkörper (16) gelenkig verbindende Gelenkeinrichtung (22),

wobei die Gelenkeinrichtung (22) eine Aktorik (52) aufweist, die mittels einer Steuereinrichtung (38) derart aktiv ansteuerbar ist, dass die Lage des Auftrittkörpers (16) relativ zum Prothesenschaft (12) veränderbar ist, und die Prothesenvorrichtung (10) ferner wenigstens ein Sensorelement (26, 28, 30, 32, 34, 36, 41) aufweist, das zur berührungsfreien Erfassung der Beschaffenheit des Untergrunds ausgebildet ist, sowie eine Bewegungserfassungseinrichtung (40), die Bewegungsparameter bezüglich der aktuellen Bewegung der Prothesenvorrichtung (10), insbesondere bezüglich einer Rotation und Translation der Prothesenvorrichtung (10), erfasst, umfassend die Schritte:

- berührungsfreies Erfassen der Beschaffenheit des Untergrunds mittels des wenigstens einen Sensorelements,
- Auswerten der von dem wenigstens einen Sensorelement (26, 28, 30, 32, 34, 36, 41) bereitgestellten Information hinsichtlich der Beschaffenheit des Untergrunds und
- Ansteuern der Aktorik (52) der Gelenkeinrichtung (22) nach Maßgabe der von dem Sensorelement (26, 28, 30, 32, 34, 36, 41) hinsichtlich der Beschaffenheit des Untergrunds ausgegebenen Information, wobei die von dem wenigstens einen Sensorelement (26, 28, 30, 32, 34, 36, 41) erfasste Information in Relation mit den von der Bewegungserfassungseinrichtung (40) erfassten Bewegungsparametern gebracht wird, derart, dass sie in ein globales Bezugssystem transformiert werden.

**12.** Verfahren nach Anspruch 11,
**dadurch gekennzeichnet, dass** die Prothesenvorrichtung (10) eine Projektionseinrichtung aufweist, wobei mit der Projektionseinrichtung ein optisch erfassbares Muster, insbesondere ein Gitter, auf den Untergrund projiziert wird, wobei die Istform des auf den Untergrund projizierten optischen Musters erfasst wird und mit einer Sollform verglichen wird und anhand der Abweichung zwischen der Istform und der Sollform die Beschaffenheit des Untergrunds, insbesondere dessen Geometrie, ermittelt wird.

**13.** Verfahren nach Anspruch 11 oder 12,
**dadurch gekennzeichnet, dass** die von der Bewegungserfassungseinrichtung (40) erfassten Bewegungsparameter in Kombination mit der von dem wenigstens einen Sensorelement (26, 28, 30, 32, 34, 36, 41) erfassten Information bezüglich der Beschaffenheit des Untergrunds ausgewertet werden, dass anhand des Auswertungsergebnisses ein Modell, insbesondere ein dreidimensionales Modell, des Untergrunds ermittelt wird und dass nach Maßgabe des ermittelten Modells des Untergrunds die Aktorik (52) der Prothesenvorrichtung (10) angesteuert wird.

**14.** Verfahren nach einem der Ansprüche 11 bis 13,
**dadurch gekennzeichnet, dass** die Transformation in das globale Bezugssystem mittels Quaternionenrotation erfolgt.

**15.** Verfahren nach einem der Ansprüche 11 bis 14,
**dadurch gekennzeichnet, dass** eine Plausibilitätskontrolle hinsichtlich der über eine Mehrzahl von Sensorelementen (26, 28, 30, 32, 34, 36, 41) ermittelten Informationen über die Beschaffenheit des Untergrunds durchgeführt wird, wobei nicht-plausible Informationen verworfen werden.

**16.** Verfahren nach einem der Ansprüche 11 bis 15,
**dadurch gekennzeichnet, dass** bei der Ermittlung der Beschaffenheit des Untergrunds Navigationsdaten eines Navigationssystems berücksichtigt werden.

**Claims**

**1.** A prosthetic device (10), especially a foot prosthesis or leg prosthesis, comprising:

- a prosthesis shaft (12), which can be coupled to a leg of a patient to be attended to,
- at least one treading body (16) for contacting a ground (U), and
- at least one joint device (22) hingedly connecting the prosthesis shaft (12) to the treading body (16),
- wherein the joint device (22) comprises an actuator (52) which can be actively controlled by a controller (38) in such a manner that the position of the treading body (16) relative to the prosthesis shaft (12) can be changed,
- and wherein the prosthetic device (10) further comprises at least one sensor element (26, 28, 30, 32, 34, 36, 41), which is configured for contact-free detection of the condition of the ground, wherein the actuator (52) of the joint device (22) can be controlled in accordance with the information regarding the condition of the ground (U) detected in a contact-free manner by the sensor element (26, 28, 30, 32, 34, 36, 41), and at least one motion detection device (40) configured for detecting motion parameters regarding the current movement of the prosthetic device (10), especially regarding rotation and translation of the prosthetic device (10),
- **characterized in that** the controller (38) is configured for evaluating the motion parameters detected by the motion detection device (40) in combination with the information regarding the condition of the ground detected by the at least one sensor element (26, 28, 30, 32, 34, 36, 41) in order to determine a model of the ground, according to which the actuator (52) of the prosthetic device (10) can be adjusted, wherein the current position of the prosthetic device (10) in space with respect to a global coordinate system is determined from the motion parameters, and the detected information regarding the ground (11) and the condition thereof is then assigned to this global coordinate system.

**2.** The prosthetic device (10) according to claim 1,
**characterized in that** the sensor element (26, 28, 30, 32, 34, 36, 41) comprises a camera or a distance measuring sensor or a laser scanner.

**3.** The prosthetic device (10) according to claims 1 or 2,
**characterized in that** a plurality of sensor elements are arranged on the treading body (16).

**4.** The prosthetic device (10) according to any one of the preceding claims, **characterized in that** two sensor elements, especially two cameras, can in each case be combined for detecting a depth image of the ground.

**5.** The prosthetic device (10) according to any one of the preceding claims, **characterized in that** a plurality of sensor elements (26, 28, 30, 32, 34, 36, 41) are arranged on the treading body (16) on the sole thereof facing the ground.

**6.** The prosthetic device (10) according to any one of the preceding claims, **characterized in that** a plurality of sensor elements (26, 28, 30, 32, 34, 36, 41) are arranged on the treading body (16) on the front side above the sole facing the ground.

7. The prosthetic device (10) according to any one of the preceding claims, **characterized by** a projection device (36) arranged on the treading body (16), by means of which an optically detectable pattern, especially a grid, can be projected onto the ground, wherein the condition of the ground can be determined from the shape of the optically detectable pattern projected onto the ground.

8. The prosthetic device (10) according to any one of the preceding claims, **characterized in that** the motion detection device (40) comprises at least one rotation sensor and/or at least one inertial sensor.

9. The prosthetic device (10) according to any one of the preceding claims, **characterized in that** the motion detection device (40) is arranged on or integrated in the prosthesis shaft (12) or the treading body (16) of the prosthetic device (10).

10. The prosthetic device (10) according to any one of the preceding claims, **characterized by** at least one fusion filter which can be supplied with signals for plausibility checking representing the information regarding the ground detected by the at least one sensor element (26, 28, 30, 32, 34, 36, 41) and the motion parameters of the motion detection device (40).

11. A method for controlling or regulating a prosthetic device (10), in particular according to any one of the preceding claims, the prosthetic device (10) comprising:

- a prosthesis shaft (12), which can be coupled to a leg of a patient to be attended to,
- at least one treading body (16) for contacting a ground, and
- at least one joint device (22) hingedly connecting the prosthesis shaft (12) to the treading body (16),
- wherein the joint device (22) comprises an actuator (52) which can be actively controlled by a controller (38) in such a manner that the position of the treading body (16) relative to the prosthesis shaft (12) can be changed,
- and wherein the prosthetic device (10) further comprises at least one sensor element (26, 28, 30, 32, 34, 36, 41), which is configured for contact-free detection of the condition of the ground, and at least one motion detection device (40) detecting motion parameters regarding the current movement of the prosthetic device (10), especially regarding rotation and translation of the prosthetic device (10), comprising the steps:

- detecting the condition of the ground by means of the at least one sensor element in a contact-free manner,
- evaluating the information regarding the condition of the ground provided by the at least one sensor element (26, 28, 30, 32, 34, 36, 41), and
- controlling the actuator (52) of the joint device (22) in accordance with the information regarding the condition of the ground output by the sensor element (26, 28, 30, 32, 34, 36, 41), wherein the information detected by the at least one sensor element (26, 28, 30, 32, 34, 36, 41) is related to the motion parameters detected by the motion detection device (40) in such a manner that they are transformed into a global reference system.

12. The method according to claim 11,
**characterized in that** the prosthetic device (10) comprises a projection device, wherein an optically detectable pattern, especially a grid, is projected onto the ground by the projection device, wherein the actual shape of the optical pattern projected onto the ground is detected and compared to a nominal shape, and the condition of the ground, especially the geometry thereof, is determined from the deviation between the actual shape and the nominal shape.

13. The method according to claims 11 or 12,
**characterized in that** the motion parameters detected by the motion detection device (40) are evaluated in combination with the information regarding the condition of the ground detected by the at least one sensor element (26, 28, 30, 32, 34, 36, 41), **in that** a model, especially a three-dimensional model, of the ground is determined from the evaluation result, and **in that** the actuator (52) of the prosthetic device (10) is controlled in accordance with the determined model of the ground.

14. The method according to any one of claims 11 to 13,
**characterized in that** the transformation into the global reference system is effected by means of quaternion rotation.

**15.** The method according to any one of claims 11 to 14,
**characterized in that** a plausibility check is performed with respect to the information regarding the condition of the ground detected by a plurality of sensor elements (26, 28, 30, 32, 34, 36, 41), wherein non-plausible information is discarded.

**16.** The method according to any one of claims 11 to 15,
**characterized in that** navigation data of a navigation system is taken into consideration when determining the condition of the ground.

**Revendications**

**1.** Dispositif prothétique (10), en particulier prothèse de pied ou prothèse de jambe, comprenant :

- une tige de prothèse (12) pouvant être accouplée à la jambe d'un patient à traiter,
- au moins un corps de marche (16) venant en contact avec un terrain (U), et
- au moins un dispositif d'articulation (22) raccordant de manière articulée la tige de prothèse (12) au corps de marche (16),

le dispositif d'articulation (22) présentant un système d'actionneurs (52) pouvant être commandé activement au moyen d'un dispositif de commande (38) de manière à permettre la modification de la position du corps de marche (16) par rapport à la tige de prothèse (12),
et le dispositif prothétique (10) comprenant en outre au moins un élément capteur (26, 28, 30, 32, 34, 36, 41) qui est conçu pour détecter sans contact la nature du terrain, le système d'actionneurs (52) du dispositif d'articulation (22) pouvant être commandé en fonction de l'information relative à la nature du terrain (U) détectée sans contact par l'élément capteur (26, 28, 30, 32, 34, 36, 41), ainsi qu'au moins un dispositif de détection de mouvement (40) qui est conçu pour détecter des paramètres de mouvement relatifs au mouvement actuel du dispositif prothétique (10), en particulier relatifs à une rotation et une translation du dispositif prothétique (10), **caractérisé en ce que** le dispositif de commande (38) est conçu pour évaluer les paramètres de mouvement détectés par le dispositif de détection de mouvement (40) en combinaison avec l'information relative à la nature du terrain détectée par ledit au moins un élément capteur (26, 28, 30, 32, 34, 36, 41), afin de déterminer un modèle du terrain en fonction duquel le système d'actionneurs (52) du dispositif prothétique (10) est réglable, les paramètres de mouvement étant utilisés pour déterminer la position actuelle du dispositif prothétique (10) dans l'espace par rapport à un système de coordonnées global et l'information détectée sur le terrain (11) et sa nature étant ensuite associée à ce système de coordonnées global.

**2.** Dispositif prothétique (10) selon la revendication 1,
**caractérisé en ce que** l'élément capteur (26, 28, 30, 32, 34, 36, 41) comprend une caméra ou un capteur de mesure de distance ou un scanner laser.

**3.** Dispositif prothétique (10) selon la revendication 1 ou 2,
**caractérisé en ce qu'**une pluralité d'éléments capteurs est disposée sur le corps de marche (16).

**4.** Dispositif prothétique (10) selon l'une des revendications précédentes,
**caractérisé en ce que** deux éléments capteurs, en particulier deux caméras, respectivement, peuvent être combinés pour détecter une image en relief du terrain.

**5.** Dispositif prothétique (10) selon l'une des revendications précédentes, **caractérisé en ce qu'**une pluralité d'éléments capteurs (26, 28, 30, 32, 34, 36, 41) est disposée sur le corps de marche (16), sur sa semelle tournée vers le terrain.

**6.** Dispositif prothétique (10) selon l'une des revendications précédentes, **caractérisé en ce qu'**une pluralité d'éléments capteurs (26, 28, 30, 32, 34, 36, 41) est disposée sur le corps de marche (16), sur la face frontale au-dessus de la semelle tournée vers le terrain.

**7.** Dispositif prothétique (10) selon l'une des revendications précédentes, **caractérisé par** un dispositif de projection (36) disposé sur le corps de marche (16), au moyen duquel un motif détectable optiquement, en particulier un réseau, est susceptible d'être projeté sur le terrain, la nature du terrain pouvant être déterminée à partir de la forme du motif détectable optiquement projeté sur le terrain.

8. Dispositif prothétique (10) selon l'une des revendications précédentes, **caractérisé en ce que** le dispositif de détection de mouvement (40) comprend au moins un capteur de rotation ou/et au moins un capteur d'inertie.

9. Dispositif prothétique (10) selon l'une des revendications précédentes, **caractérisé en ce que** le dispositif de détection de mouvement (40) est disposé sur la tige de prothèse (12) ou le corps de marche (16) du dispositif prothétique (10) ou intégré dans celle ou celui-ci.

10. Dispositif prothétique (10) selon l'une des revendications précédentes, **caractérisé par** au moins un filtre de fusion auquel peuvent être amenés pour un contrôle de plausibilité des signaux qui représentent l'information relative au terrain déterminée par ledit au moins un élément capteur (26, 28, 30, 32, 34, 36, 41) ainsi que les paramètres de mouvement du dispositif de détection de mouvement (40).

11. Procédé de commande et de régulation d'un dispositif prothétique (10), en particulier selon l'une des revendications précédentes, le dispositif prothétique (10) comprenant :

   - une tige de prothèse (12) pouvant être accouplée à la jambe d'un patient à traiter,
   - au moins un corps de marche (16) venant en contact avec un terrain, et
   - au moins un dispositif d'articulation (22) raccordant de manière articulée la tige de prothèse (12) au corps de marche (16),

   le dispositif d'articulation (22) présentant un système d'actionneurs (52) pouvant être commandé activement au moyen d'un dispositif de commande (38) de manière à permettre la modification de la position du corps de marche (16) par rapport à la tige de prothèse (12),
   et le dispositif prothétique (10) comprenant en outre au moins un élément capteur (26, 28, 30, 32, 34, 36, 41) qui est conçu pour détecter sans contact la nature du terrain, ainsi qu'un dispositif de détection de mouvement (40) qui détecte des paramètres de mouvement relatifs au mouvement actuel du dispositif prothétique (10), en particulier relatifs à une rotation et une translation du dispositif prothétique (10),
   ledit procédé comprenant les étapes de :

   - détection sans contact de la nature du terrain au moyen dudit au moins un élément capteur,
   - évaluation de l'information relative à la nature du terrain fournie par ledit au moins un élément capteur (26, 28, 30, 32, 34, 36, 41) et
   - commande du système d'actionneurs (52) du dispositif d'articulation (22) en fonction de l'information relative à la nature du terrain fournie par l'élément capteur (26, 28, 30, 32, 34, 36, 41), l'information détectée par ledit au moins un élément capteur (26, 28, 30, 32, 34, 36, 41) étant mise en relation avec les paramètres de mouvement détectés par le dispositif de détection de mouvement (40) de manière à les transformer en un système de référence global.

12. Procédé selon la revendication 11, **caractérisé en ce que** le dispositif prothétique (10) comprend un dispositif de projection, un motif détectable optiquement, en particulier un réseau, étant projeté sur le terrain au moyen du dispositif de projection, la forme réelle du motif optique projeté sur le terrain étant détectée et comparée avec une forme théorique et la nature du terrain, en particulier sa géométrie, étant déterminée à partir de la différence entre la forme réelle et la forme théorique.

13. Procédé selon la revendication 11 ou 12, **caractérisé en ce que** les paramètres de mouvement détectés par le dispositif de détection de mouvement (40) sont évalués en combinaison avec l'information relative à la nature du terrain détectée par ledit au moins un élément capteur (26, 28, 30, 32, 34, 36, 41), **en ce qu'**un modèle, en particulier un modèle tridimensionnel, du terrain est déterminé à partir du résultat de l'évaluation et **en ce que** le système d'actionneurs (52) du dispositif prothétique (10) est commandé en fonction du modèle déterminé du terrain.

14. Procédé selon l'une des revendications 11 à 13, **caractérisé en ce que** la transformation en un système de référence global est réalisée au moyen d'une rotation quaternionique.

15. Procédé selon l'une des revendications 11 à 14, **caractérisé en ce qu'**un contrôle de plausibilité concernant les informations sur la nature du terrain déterminées par une pluralité d'éléments capteurs (26, 28, 30, 32, 34, 36, 41) est effectué, les informations non plausibles étant rejetées.

16. Procédé selon l'une des revendications 11 à 15,
**caractérisé en ce que** les données de navigation d'un système de navigation sont prises en compte dans la détermination de la nature du terrain.

Fig. 2

Fig. 1

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006083913 A2
- US 20090069898 A1
- US 6755870 B
- EP 1933775 A2
- EP 1942843 A2
- EP 1531767 B1
- EP 2036518 A1